# EUROPEAN PATENT APPLICATION

(11) **EP 3 881 764 A1**
(43) Date of publication of application: **22.09.2021**
(21) Application number: 18940272.0
(22) Date of filing: 13.11.2018
(51) Int. Cl.: A61B 5/16, A61B 10/00

(54) **PERIPHERAL NERVE EXAMINATION DEVICE, PERIPHERAL NERVE EXAMINATION METHOD, AND PROGRAM**

(71) Applicant: Nihon Kohden Corporation, Tokyo 161-8560 (JP)
(72) Inventor: KAIAMI Takashi, Tokorozawa-shi, Saitama 359-0037 (JP); YOSHIDA Ryoko, Tokorozawa-shi, Saitama 359-0037 (JP); MOTOGI Jun, Tokorozawa-shi, Saitama 359-0037 (JP)
(74) Representative: Grünecker Patent- und Rechtsanwälte PartG mbB
(86) International application number: PCT/JP2018/041959
(87) International publication number: WO 2020/100209

(57) **Abstract**

A peripheral nerve examination device (1) evaluates a nerve of a subject on the basis of an expression by the subject of perception of stimulation. A stimulation control unit (13) controls a plurality of stimulations of varying intensity that are imparted to the subject. A measurement unit (12) obtains a measured value of the reaction time of the subject to each of the plurality of stimulations. An output unit (14) outputs a relationship between the measured values and the intensity of the plurality of stimulations.

## Description

### TECHNICAL FIELD

The present disclosure relates to a peripheral nerve examination device, a peripheral nerve examination method, and a program that examine a peripheral nerve of a subject.

### BACKGROUND ART

Patent Literature 1 is known as a technique for examining a peripheral nerve with a simple configuration. Patent Literature 1 discloses a pain sensory nerve stimulation device that can selectively stimulate only C fibers.

A peripheral nerve examination device disclosed in Non-Patent Literature 1 is a device to which the technique disclosed in Patent Literature 1 is applied. In an examination using the peripheral nerve examination device, a stimulation electrode is attached to a subject, the subject holds a switch, and an examiner operates the peripheral nerve examination device to set a current value to be output and output a stimulation. When the subject feels a stimulation, the subject presses the switch. The peripheral nerve examination device examines normality of a peripheral nerve of the subject based on a current value or reaction time (time from when the stimulation is started to when the switch is pressed). When such a peripheral nerve examination device is used, a peripheral nerve examination can be performed with a simple configuration without attaching an electrode to a scalp as in a case of an electroencephalogram examination.

### CITATION LIST

### PATENT LITERATURE

Patent Literature 1: JP2011-164879A

### NON-PATENT LITERATURE

Non-Patent Literature 1: Portable Peripheral Nerve Examination Device PNS-7000, [searched on Sept. 25, 2017], Internet <URL: http://www.nihonkohden.co.jp/iryo/documents/pdf/H901653C.pdf>
Non-Patent Literature 2: Jun Kimura, Nobuo Kouhara, "Nerve conduction Studies and Electromyography", Igaku-Shoin Ltd.
Non-Patent Literature 3: Koji Inui, Ryusuke Kakigi, "Pain perception in humans: use of intraepidermal electrical stimulation", J Neurol Neurosurg Psychiatry 83: 551-556, 2012
Non-Patent Literature 4: Maxim Churyukanov, Leon Plaghki, Valery Legrain, Andre Mouraux, "Thermal Detection Thresholds of Aδ- and C-Fibre Afferents Activated by Brief Co2 Laser Pulses Applied onto the Human Hairy Skin", PLoS ONE 7(4): e35817. https://doi.org/10.1371/journal.pone.0035817
Non-Patent Literature 5: Mark J. Zylka, Frank L. Rice, and David J. Anderson, "Topographically Distinct Epidermal Nociceptive Cricuits Revealed by Axonal Tracers Targeted to Mrgprd", Neuron, Vol. 45, 17-25, January 6, 2005

### SUMMARY OF INVENTION

### TECHNICAL PROBLEM

In an examination using a device such as the device disclosed in Non-Patent Literature 1, reaction time of the subject is measured for each of a plurality of stimulations while intensities of the stimulations are changed. In general, a nervous system of the subject is examined based on a minimum stimulation intensity that the subject can sense and reaction time.

In such a peripheral nerve examination, reaction time for a stimulation is determined in accordance with the pressing of the switch by the subject (expression of sensing). As a result, the subject may make an operation error, or a method for performing an examination on the subject may be inappropriate. In such a case, reaction time of each of the plurality of stimulations with varying intensities is not appropriate, but a minimum sensed current value may be a normal value on occasion. As a result, it may be determined that an examination for the subject is performed normally. That is, there is a problem that whether an examination is performed normally is not known in an examination in which only a minimum sensed stimulation intensity is used a determination criterion.

A main object of the present disclosure is to provide a peripheral nerve examination device, a peripheral nerve examination method, and a program that can determine whether an examination is performed normally when a peripheral nerve of a subject is examined based on an expression (for example, pressing a switch) of sensing a stimulation by the subject.

### SOLUTION TO PROBLEM

A peripheral nerve examination device according to one aspect of the present disclosure is a device that examines a peripheral nerve of a subject based on an expression of sensing a stimulation by the subject. The peripheral nerve examination device includes
a stimulation control unit that applies a plurality of stimulations of varying intensities to the subject,
a measurement unit that obtains a measured value of reaction time of the subject for each of the plurality of stimulations, and
an output unit that outputs a relationship between a change in the intensities of the stimulations and a change in the measured values.

The peripheral nerve examination device is configured to output a relationship between a change in the intensities of the stimulations and a change in the measured values of the reaction time. An examiner can easily determine whether an examination is performed normally (whether an examination of a peripheral nerve is performed normally) by referring to output information.

### BRIEF DESCRIPTION OF DRAWINGS

FIG. 1 is a block diagram illustrating a configuration of a peripheral nerve examination device 1 according to a first embodiment.
FIG. 2 illustrates an example of an output screen of the output unit 14 (during a normal examination) according to the first embodiment.
FIG. 3 illustrates an example of an output screen of the output unit 14 (during an abnormal examination) according to the first embodiment.
FIG. 4 illustrates an example of an output screen of the output unit 14 (during a normal examination) according to the first embodiment.
FIG. 5 is a block diagram illustrating a configuration of a peripheral nerve examination device 1 according to a second embodiment.
FIG. 6 is a diagram illustrating a hardware configuration of the peripheral nerve examination device 1A according to the first or second embodiment.

### DESCRIPTION OF EMBODIMENTS

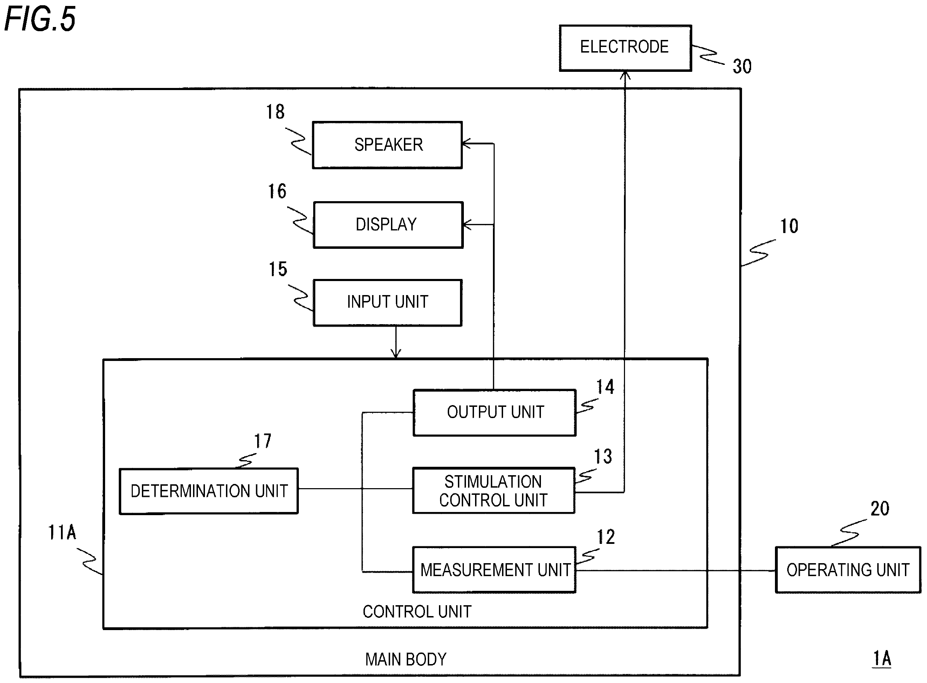

First, the premise of the presently disclosed subject matter will be described in detail. There are a plurality of types of nerve fibers (C fibers, Aδ fibers, and the like). It is known that the C fibers are more easily excited by an electrical stimulation than the Aδ fibers (see Non-Patent Literature 3 and Non-Patent Literature 4). For example, Non-Patent Literature 3 discloses that the C fibers react to a stimulation by a laser at about 40°C and the Aδ fibers react to a stimulation at about 46°C. That is, the C fibers react to a stimulation having a lower intensity compared with the Aδ fibers. This is because a nerve of the C fiber extends to the periphery more than a nerve of the Aδ fiber (see FIG. 5B of Non-Patent Literature 5), and the C fiber reacts to a weak stimulation.

It is widely known that a conduction time varies depending on the types of nerve fibers (see Non-Patent Literature 2). For example, a conduction speed of the Aδ fiber is about 10 m/s to 30 m/s, and a conduction speed of the C fiber is about 0.5 m/s to 2.5 m/s.

That is, when a stimulation having a low intensity (0.05 mA to 0.20 mA in the following example) is applied to a subject, only the C fibers react to the stimulation, and a conduction speed of the stimulation is slow. That is, time from when the stimulation is applied to when the stimulation is sensed is long.

When a stimulation having a high intensity (0.30 mA or higher in the following example) is applied to a subject, the Aδ fibers also react to the stimulation, and thus a conduction speed of the stimulation is fast. That is, time from when the stimulation is applied to when the stimulation is sensed is short.

A peripheral nerve examination device 1 to be described below is operated based on these characteristics.

### First Embodiment

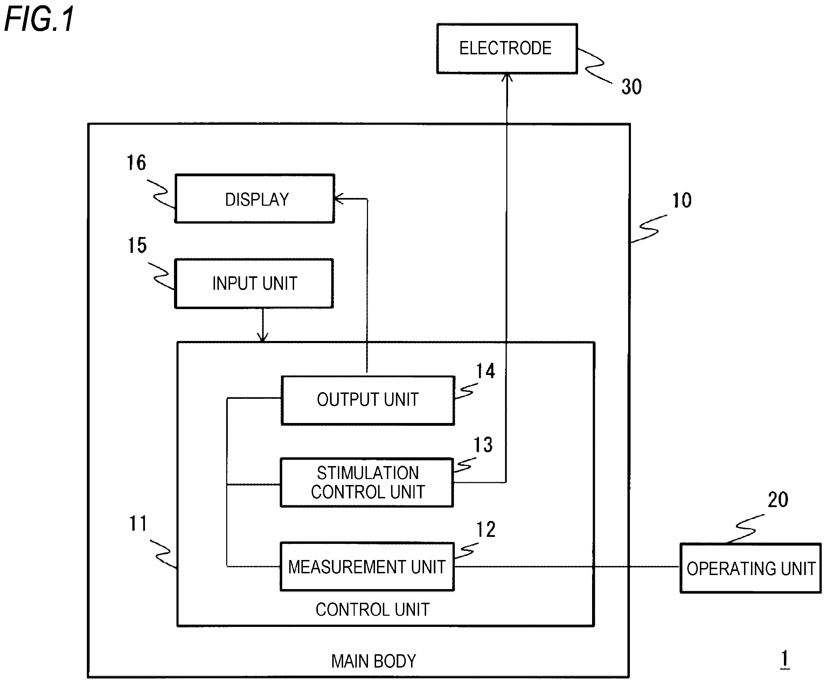

The peripheral nerve examination device 1 according to the present embodiment has a configuration to which the characteristics described above are applied. Hereinafter, embodiments of the presently disclosed subject matter will be described with reference to the drawings. FIG. 1 is a block diagram illustrating a configuration of the peripheral nerve examination device 1 according to the present embodiment.

The peripheral nerve examination device 1 is a device that evaluates a peripheral nerve of a subject based on an expression of sensing a stimulation by the subject. More specifically, the peripheral nerve examination device 1 applies a stimulation to a peripheral nerve of the subject via an electrode 30. When the subject senses the stimulation, the subject operates an operating unit 20 (preferably, a holding type switch) to express that the subject senses the stimulation. The peripheral nerve examination device 1 evaluates a peripheral nerve of the subject based on an intensity of the applied stimulation and a measured value of reaction time (time from when the stimulation is started to when the operating unit 20 is operated). Hereinafter, a detailed configuration will be described.

The peripheral nerve examination device 1 can include a main body 10 and the operating unit 20. The main body 10 and the operating unit 20 are connected by a general cable. The main body 10 is attachable to and detachable from the disposable electrode 30. The electrode 30 is an example of a member that applies a stimulation to a peripheral nerve of the subject, and is, for example, a disposable electrode that can be attached to a skin of the subject. The same as or similar to Patent Literature 1, the electrode 30 may include a first electrode that is used by slightly piercing a tip of the first electrode into the skin of the subject, and a second electrode that is disposed around the first electrode and is used by being brought into contact with the skin of the subject.

Instead of the electrode 30, a probe or the like that applies a stimulation to a peripheral nerve of the subject may be connected to the main body 10.

The main body 10 is preferably a housing having a size that can be held by an examiner (for example, a medical worker). An input unit 15 and a display 16 are disposed on the housing of the main body 10. For example, the input unit 15 is a button, a scroll wheel, or the like provided on the housing of the main body 10. The examiner operates the input unit 15 to input a setting, a signal to start a stimulation, and the like. The display 16 is a liquid crystal display provided on the housing of the main body 10. The main body 10 may also include a speaker or a power source (not illustrated). The display 16 may have a function of the input unit 15 (a so-called touch display).

The operating unit 20 is an interface operated by the subject whose peripheral nerve is to be examined. The operating unit 20 is preferably a holding type switch to be held by the subject. Various electric circuits and the like for transmitting a detection signal to the main body 10 when the switch is pressed are provided inside the operating unit 20. The detection signal is input to the main body 10 via a cable.

When the subject senses a stimulation via the electrode 30, the subject operates the operating unit 20 to notify the main body 10 that the stimulation is sensed (in other words, the subject expresses that the stimulation is sensed).

The main body 10 includes a control unit 11. The control unit 11 is a processing unit that executes various kinds of controls of the peripheral nerve examination device 1. The control unit 11 can include a measurement unit 12, a stimulation control unit 13, and an output unit 14.

The stimulation control unit 13 controls an intensity and timing of a stimulation applied via the electrode 30. The stimulation control unit 13 applies a plurality of stimulations of varying intensities to the subject via the electrode 30. An intensity of a stimulation may be controlled by a magnitude of a current value or a voltage value. The intensity of the stimulation is controlled by a change in a current value in the following description.

A change in the intensity of the stimulation and the number of stimulations controlled by the stimulation control unit 13 can be set by the examiner via the input unit 15. The examiner inputs, for example, a current value of a first stimulation, an increase and decrease unit of the current value, the number of stimulations, and the like via the input unit 15. The examiner may input a current value and stimulation timing for each stimulation via the input unit 15. The stimulation control unit 13 executes a plurality of electric stimulations via the electrode 30 based on information such as the input current value. The stimulation control unit 13 may control an output of the stimulation such that an intensity gradually increases, or may control an output of the stimulation such that an intensity gradually decreases.

The measurement unit 12 obtains a measured value of reaction time for each of the plurality of stimulations applied via the electrode 30. The measurement unit 12 may calculate a measured value of reaction time for each stimulation by using a notification signal indicating the start of a stimulation by the stimulation control unit 13, a detection signal received from the operating unit 20, and the like.

The output unit 14 acquires the measured value of the reaction time for each stimulation measured by the measurement unit 12. The output unit 14 acquires information on an intensity of each stimulation controlled by the stimulation control unit 13. That is, the output unit 14 acquires a set of an intensity and a measured value of reaction time for each stimulation.

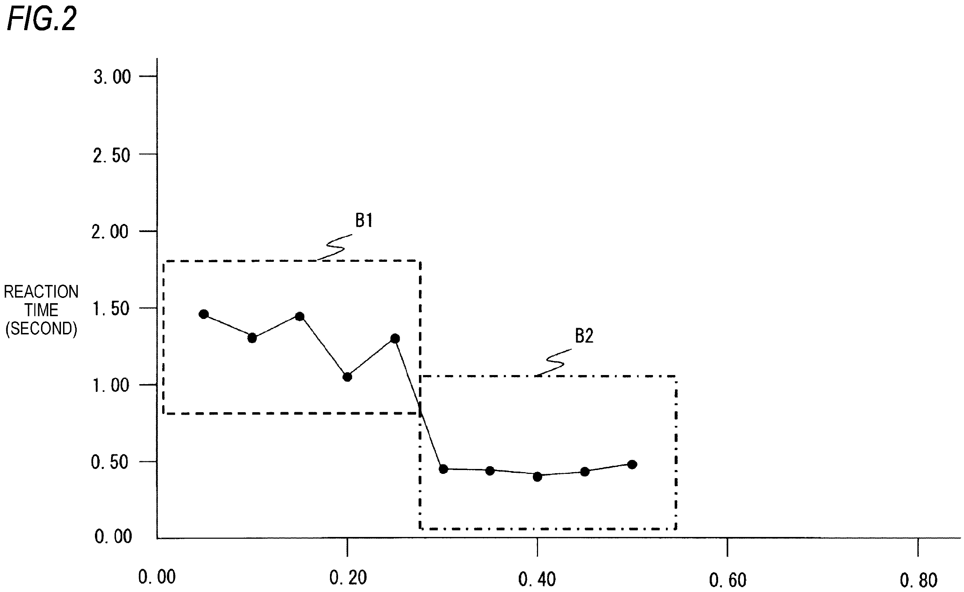

The output unit 14 outputs a relationship between the intensity of each stimulation and the measured value of the reaction time for each of the plurality of stimulations. The output unit 14 preferably outputs data for displaying, in a two-dimensional graph on the display 16, the relationship between the intensity of the stimulation and the measured value of the reaction time of the stimulation. Such a display will be described with reference to FIGS. 2 to 4.

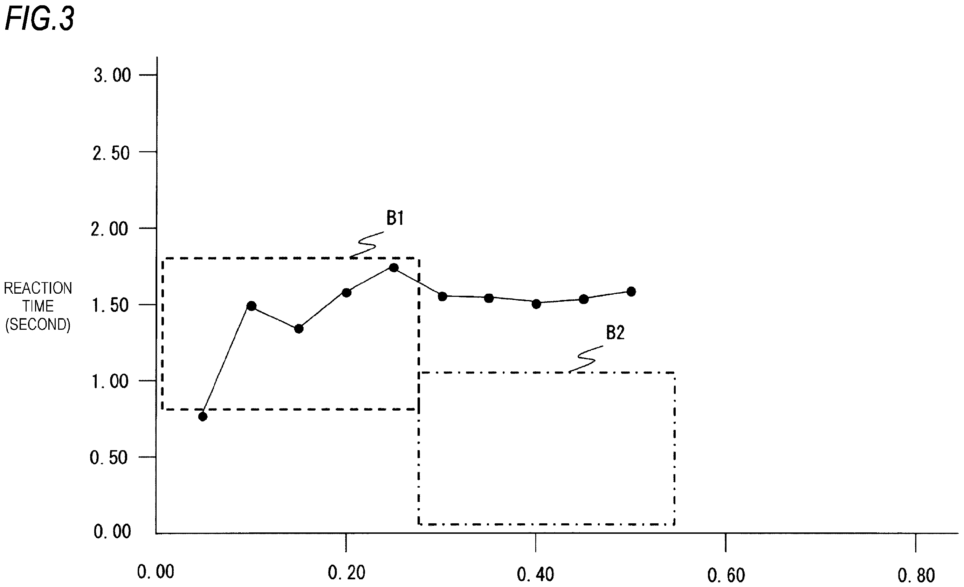

FIG. 2 illustrates a two-dimensional graph when a peripheral nerve of the subject is examined normally. FIG. 3 illustrates a two-dimensional graph when a peripheral nerve of the subject is not examined normally. A horizontal axis (first axis) of the two-dimensional graph indicates an intensity (current value) of a stimulation and a vertical axis (second axis) indicates reaction time. Alternatively, the horizontal axis may indicate reaction time, and the vertical axis may indicate an intensity (current value) of a stimulation.

As illustrated in the drawing, the output unit 14 plots current values of stimulations and measured values of reaction time, and outputs data for a display in which points are connected by a line. As described above, when an intensity of a stimulation is low (equal to or lower than a predetermined threshold), only the C fibers having a low conduction speed react, and when an intensity of a stimulation is high (equal to or higher than the predetermined threshold), the Aδ fibers having a high conduction speed also react. Therefore, as illustrated in FIG. 2, when an intensity of a stimulation is equal to or higher than a certain value, it is normal that the reaction time rapidly decreases. In the example in FIG. 2, the reaction time is about 1.00 seconds to 1.50 seconds before the current value reaches about 0.20 mA, and the reaction time is about 0.50 seconds or less after the current value exceeds 0.30 mA.

Here, the output unit 14 preferably outputs index information indicating at least one of a normal range and an abnormal range of the reaction time. In the example in FIG. 2, the output unit 14 outputs data for displaying a box B1 (first box) that is a region (first region) indicating a normal range of reaction time of the C fibers and a box B2 (second box) that is a region (second region) indicating a normal range of reaction time of the Aδ fibers. Sizes of the box B1 and the box B2 (a range of the reaction time and a range of the current value) may be determined based on, for example, examination results of a sufficient number of subjects. Alternatively, the sizes of the boxes B1 and B2 may be determined in consideration of attributes (age, sex, and the like) of a subject to be examined. Whether measured values fall within ranges of the box B1 and the box B2 is an index for determining whether an examination is performed normally.

It is desirable that the box B1 and the box B2 are indicated with different display effects. For example, the box B1 and the box B2 are preferably indicated by frame lines of different line types, frame lines of different line colors, or different colors. An examiner can easily understand a difference between stimulated nerves by indicating the box B1 and the box B2 with different display effects.

The examiner refers to the two-dimensional graph and determines whether an examination is performed normally. When measured values of reaction time rapidly decrease at a time point when a current value exceeds a certain value, the examiner determines that the examination is performed normally (FIG. 2). When measured values of reaction time do not change so much regardless of the magnitude of a current value, the examiner determines that the examination is not performed normally (abnormally) (FIG. 3). The examiner also determines that an examination is not performed normally when measured values of reaction time increase as a current value increases. In an example in FIG. 3, since measured values of reaction time are fairly large when a current value is a value at which the Aδ fibers are stimulated, it is likely that the subject does not understand an intention of an examination. When the examination is not performed normally, the examiner may perform the examination again, explain the examination, or the like. When such a determination is made, it is more preferable that the examiner appropriately refers to the box B1 and the box B2 that are indices of normal values. That is, when measured values (plots) fall within the box B1 and the box B2, the examiner may determine that the examination is likely to be performed normally.

The box B1 and the box B2 illustrated in FIG. 2 or 3 are a type of index information for determining whether reaction time is normal or abnormal. The output unit 14 may output data for displaying the index information in other forms. For example, the output unit 14 illustrates a normal value range of reaction time in the example in FIG. 2. Alternatively, the output unit 14 may output data for displaying an abnormal value range instead of the normal value range. The output unit 14 may output data for displaying a figure such as a combination of the box B1 and the box B2, or may output data for displaying only a horizontal line that divides the normal value range and the abnormal value range of reaction time.

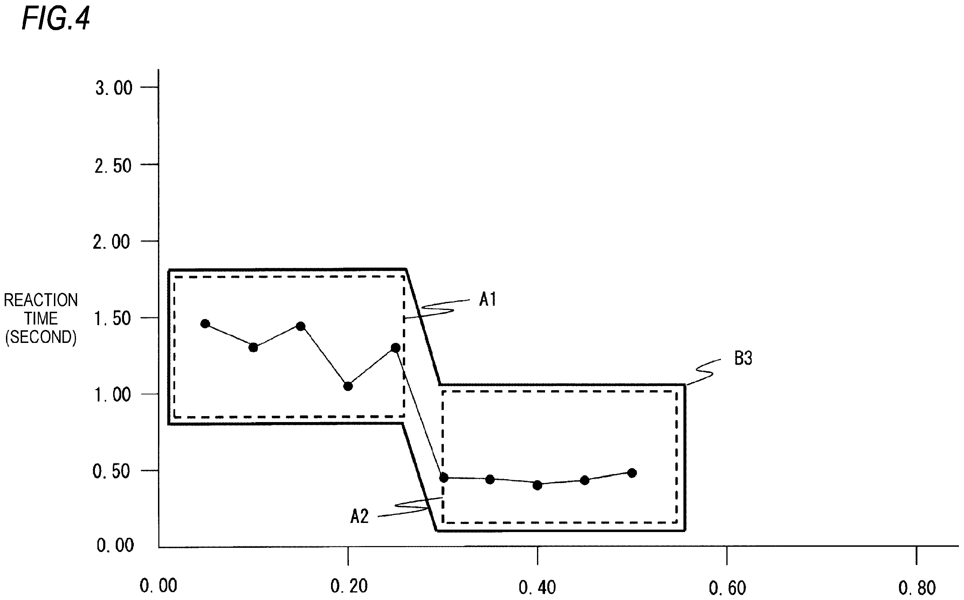

It is considered that there is a slight variation in a current value at which the Aδ fibers start to react due to a difference in subjects or a difference in stimulated positions. Therefore, the output unit 14 may display, for example, a box B3 in FIG. 4. In the box B3, a region A1 indicating a normal reaction time of the C fibers and a region A2 indicating a normal reaction time of the Aδ fibers are connected.

As described above (FIGS. 2 to 4), the output unit 14 outputs data for displaying, in a two-dimensional graph, a relationship of a change in the measured values of the reaction time and a change in the intensities of the stimulations. The presently disclosed subject matter is not necessarily limited thereto. For example, the output unit 14 may output a relationship between the intensities of stimulations and the measured values of the reaction time to the display 16 in a table format (a table listing sets of the intensities of the stimulations and the measured values of the reaction time), or may output the relationship as an electronic file in a comma-separated values (CSV) format or the like. In this case, an examiner determines whether an examination is performed normally by referring to an output table or electronic file.

When the output unit 14 outputs the relationship in a form of a table or an electronic file, it is preferable that the output unit 14 also outputs the index information described above. For example, the output unit 14 may output a normal range of reaction time when a current value is 0.40 mA as illustrated in the table, in addition to measured values of the reaction time when the current value is 0.40 mA.

It is preferable that the output unit 14 outputs a set of an intensity of a stimulation and a measured value of reaction time for each of a plurality of stimulations. Alternatively, another unit may be used as long as the unit outputs a relationship between a change in the measured values and a change in the intensities of the stimulations. That is, when a stimulation is performed ten times, the output unit 14 may output at least a relationship between intensities of two or more stimulations and measured values of reaction time. For example, it is sufficient that the output unit 14 outputs at least two values of (a measured value of reaction time at an intensity (for example, 0.15 mA) at which the C fibers are considered to react) and (a measured value of reaction time at an intensity (for example, 0.40 mA) at which the Aδ fibers are considered to react). Even with such a configuration, the examiner can determine whether an examination is performed normally.

Next, effects of the peripheral nerve examination device 1 according to the present embodiment will be described. As described above, the Aδ fibers and the C fibers differ from each other in whether the fibers are easy to react to a stimulation, and also differ from each other in a conduction speed. Therefore, when a plurality of stimulations of varying intensities are applied, an examination may not be performed normally unless reaction time according to these characteristics is measured. The peripheral nerve examination device 1 is configured to output a relationship between a change in the measured values of the reaction time and a change in the intensities of the stimulations. For example, the peripheral nerve examination device 1 displays the measured values of the reaction time and the intensities of the stimulations in a two-dimensional graph (FIGS. 2 and 3). An examiner can easily determine whether an examination is performed normally (whether a peripheral nerve examination is performed normally) by referring to the two-dimensional graph.

The peripheral nerve examination device 1 also displays a normal value range of the reaction time (boxes B1 and B2 in the example in FIG. 2) in addition to the measured values as illustrated in FIG. 2 and the like. The examiner can accurately determine whether an examination is performed normally by comparing the measured values with the normal value range.

### Second Embodiment

A peripheral nerve examination device 1A according to a second embodiment determines whether an examination is performed normally and notifies a determination result. The peripheral nerve examination device 1A according to the second embodiment will be described below in terms of differences from the peripheral nerve examination device 1 according to the first embodiment. Processing units having the same names and the same reference numerals as those in the first embodiment execute the same operations as those in the first embodiment unless otherwise specified.

FIG. 5 is a block diagram illustrating a configuration of the peripheral nerve examination device 1A according to the present embodiment. Compared with the configuration in FIG. 1, a control unit 11A further includes a determination unit 17. The determination unit 17 acquires a current value (intensity) of each stimulation controlled by the stimulation control unit 13 and a measured value of reaction time of each stimulation measured by the measurement unit 12. Then, the determination unit 17 determines whether an examination is performed normally based on a change in the measured values relative to a change in intensities of stimulations (a change in current values). The determination unit 17 notifies the output unit 14 of a determination result.

Hereinafter, a first example of a determination processing executed by the determination unit 17 will be described. The determination unit 17 reads an index indicating normality or abnormality of the reaction time relative to the intensities of the stimulations from a hard disk or the like. The index is, for example, a normal value range (information corresponding to the box B1 or the box B2) of the reaction time illustrated in FIG. 2 or 3. When measured values of the reaction time for the stimulations fall within the normal value range, the determination unit 17 determines that an examination is performed normally. On the other hand, when measured values of the reaction time for the stimulations are out of the normal value range, the determination unit 17 determines that an examination is not performed normally. That is, the determination unit 17 automatically determines whether an examination is performed normally which is visually checked by the examiner in FIGS. 2 and 3.

Next, a second example of the determination processing executed by the determination unit 17 will be described. The determination unit 17 refers to a change in measured values of reaction time when a current value is changed. Then, the determination unit 17 determines whether there is a point where the measured values of the reaction time change rapidly. More specifically, the determination unit 17 determines whether there is a point where the measured values of the reaction time rapidly decrease when intensities of the stimulations increase. When such a point is present, the determination unit 17 determines that an examination is performed normally. On the other hand, when there is no such a point where the measured values of the reaction time rapidly decrease, the determination unit 17 determines that an examination is not performed normally.

The determination unit 17 may determine whether an examination is performed normally by combining the first example and the second example described above. That is, the determination unit 17 determines that an examination is performed normally when the measured values fall within the normal value range and when there is a point where the measured values change rapidly.

The output unit 14 outputs the determination result of the determination unit 17 via the display 16 or a speaker 18. For example, the output unit 14 may display a warning message indicating that an examination is not performed normally on the display 16 or may output an alarm sound via the speaker 18. Alternatively, the output unit 14 may also appropriately perform a display (a message of "examination is ended normally") or a voice notification when an examination is performed normally.

The output unit 14 may display the two-dimensional graph or the like illustrated in FIG. 2 or 3 on the display 16.

Next, effects of the peripheral nerve examination device 1A according to the present embodiment will be described. The peripheral nerve examination device 1A according to the present embodiment mechanically (automatically) determines and notifies whether an examination is performed normally. Accordingly, it is possible to accurately know whether an examination is performed normally without being affected by the subjectivity of an examiner.

Although the present disclosure made by inventors is specifically described based on embodiments described above, the present disclosure is not limited to the embodiments described above, and it is needless to say that various modifications can be made without departing from the gist of the present disclosure.

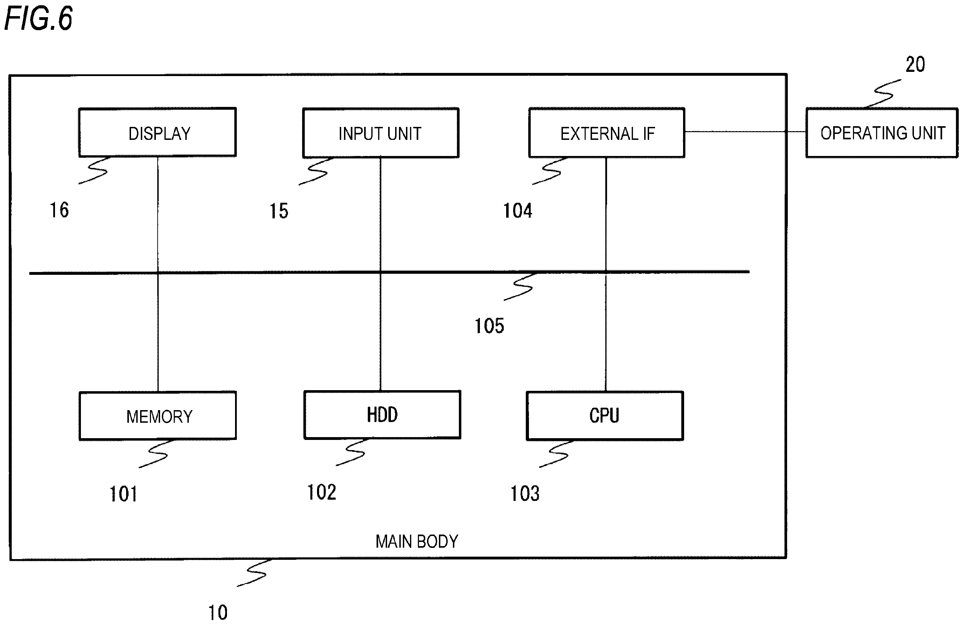

FIG. 1 and FIG. 5 are block diagrams illustrating a configuration focusing on functional characteristics of the main body 10, and FIG. 6 illustrates an example of a hardware configuration of the main body 10. The main body 10 can include one or more memories 101, a hard disk drive 102, a CPU 103, an external interface 104, the input unit 15, and the display 16. The main body 10 can include a speaker, various electric circuits, and the like (not illustrated). These components are connected to one another via a bus 105.

The external interface 104 is an interface for connecting the operating unit 20 (preferably, a switch) to the main body 10. A detection signal is input from the operating unit 20 to the external interface 104.

The hard disk 102 is a secondary storage device in the main body 10, and stores various kinds of information. The hard disk 102 does not necessarily have to be built in the main body 10, and may be detachable from the main body 10. The hard disk 102 stores various kinds of data and programs necessary for executing the operations described above.

The central processing unit (CPU) 103 loads the data or programs necessary for executing various processings of the control units 11 and 11A (the measurement unit 12, the stimulation control unit 13, and the output unit 14) on the memory 101, and executes commands included in the programs. At least a part of the various processings of the control units 11 and 11A (the measurement unit 12, the stimulation control unit 13, and the output unit 14) may be implemented by a peripheral circuit or the like (not illustrated).

Here, the program can be stored using various types of non-transitory computer readable media and can be supplied to a computer. The non-transitory computer readable media include various types of tangible storage media. Examples of the non-transitory computer readable media include a magnetic recording medium (for example, a flexible disk, a magnetic tape, and a hard disk drive), a magneto-optical recording medium (for example, a magneto-optical disk), a read only memory (CD-ROM), a CD-R, a CD-R/W, and a semiconductor memory (for example, a mask ROM, a programmable ROM (PROM), an erasable PROM (EPROM), a flash ROM, and a random access memory (RAM)). The program may be supplied to a computer by various types of transitory computer readable media. Examples of the transitory computer readable media include an electrical signal, an optical signal, and electromagnetic waves. The transitory computer readable media can supply the program to a computer via a wired communication path such as an electric wire and an optical fiber or a wireless communication path.

The present application appropriately incorporates the contents disclosed in Japanese Patent Application (Japanese Patent Application No. 2017-201671) filed on October 18, 2017.

### REFERENCE SIGNS LIST

- 1, 1A: peripheral nerve examination device
- 10: main body
- 11, 11A: control unit
- 12: measurement unit
- 13: stimulation control unit
- 14: output unit
- 15: input unit
- 16: display
- 17: determination unit
- 18: speaker
- 20: operating unit
- 30: electrode
- 101: memory
- 102: hard disk
- 103: CPU
- 104: external interface
- 105: bus

## Claims

1. A peripheral nerve examination device that examines a peripheral nerve of a subject based on an expression of sensing a stimulation by the subject, the peripheral nerve examination device comprising:
a stimulation control unit that applies a plurality of stimulations of varying intensities to the subject;
a measurement unit that obtains a measured value of reaction time of the subject for each of the plurality of stimulations; and
an output unit that outputs a relationship between a change in the intensities of the stimulations and a change in the measured values.

2. The peripheral nerve examination device according to claim 1,
wherein the output unit outputs data for displaying a two-dimensional graph in which a first axis indicates an intensity of a stimulation and a second axis indicates reaction time, and outputs data for plotting the measured value of each of the plurality of stimulations on the two-dimensional graph.

3. The peripheral nerve examination device according to claim 2,
wherein the output unit outputs data for displaying index information indicating at least one of a normal range and an abnormal range of the reaction time for an intensity of a stimulation on the two-dimensional graph.

4. The peripheral nerve examination device according to claim 3,
wherein the index information includes a first region indicating a normal or abnormal reaction time of C fibers and a second region indicating a normal or abnormal reaction time of Aδ fibers.

5. The peripheral nerve examination device according to claim 4,
wherein the first region is a first box indicating a normal reaction time of the C fibers, and
wherein the second region is a second box indicating a normal reaction time of the Aδ fibers.

6. The peripheral nerve examination device according to claim 5,
wherein the first box and the second box are indicated with different display effects.

7. The peripheral nerve examination device according to claim 6,
wherein the first box and the second box are indicated by frame lines of different colors, different types of lines, or different colors.

8. A peripheral nerve examination device that evaluates a peripheral nerve of a subject based on an expression of sensing a stimulation by the subject, the peripheral nerve examination device comprising:
a stimulation control unit that applies a plurality of stimulations of varying intensities to the subject;
a measurement unit that obtains a measured value of reaction time of the subject for each of the plurality of stimulations;
a determination unit that determines whether an examination is performed normally based on a change in the measured values relative to a change in the intensities of the stimulations; and
an output unit that outputs a determination result of the determination unit.

9. A peripheral nerve examination method for examining a peripheral nerve of a subject based on an expression of sensing a stimulation by the subject, the peripheral nerve examination method comprising:
a stimulation control step of controlling a plurality of stimulations of varying intensities that are applied to the subject;
a measurement step of measuring a measured value of reaction time of the subject for each of the plurality of stimulations; and
an output step of outputting a relationship between a change in the intensities of the stimulations and a change in the measured values.

10. A program for causing a computer to execute an examination of a peripheral nerve of a subject based on an expression of sensing a stimulation by the subject, the program implements the following steps of
a stimulation control step of controlling a plurality of stimulations of varying intensities that are applied to the subject;
a measurement step of measuring a measured value of reaction time of the subject for each of the plurality of stimulations; and
an output step of outputting a relationship between a change in the intensities of the stimulations and a change in the measured values.
